# EUROPEAN PATENT SPECIFICATION

(11) **EP 2 106 782 B1**
(45) Date of publication and mention of the grant of the patent: **06.11.2013**
(21) Application number: 09155911.2
(22) Date of filing: 23.03.2009
(51) Int. Cl.: A61K 8/02, A61Q 1/12

(54) **Process for producing powder wafers for eye shadows, face powder, make-up articles, blusher, etc. with a mosaic effect achieved by compacting variously coloured pellets**
Verfahren zur Herstellung von Pulver-Wafern für Lidschatten, Gesichtspuder, Makeup-Artikel, Rouge, usw. mit Mosaikeffekt durch Kompaktierung von verschiedenfarbigen Pellets
Processus de production de wafers en poudre pour fards à paupières, poudre pour le visage, articles de maquillage, blush, etc. avec un effet de mosaïque obtenu en compactant plusieurs pastilles colorées

(30) Priority: 31.03.2008 IT MI20080552
(43) Date of publication of application: 07.10.2009
(73) Proprietor: Intercos S.p.A., 20122 Milano (IT)
(72) Inventor: Lazzarini, Marco, 20131, MILANO (IT); Iacovacci, Francesca, 20040, CAVENAGO BRIANZA (IT)
(74) Representative: Mittler, Enrico

(56) References cited:
- JP-A- 60 067 405

## Description

The present invention relates to a process for producing powder wafers for eye shadows, face powder, make-up articles, blusher, etc. with a mosaic effect achieved by compacting variously coloured pellets.

Solid beauty products, in particular pressed powder products, are generally marketed in specific packages which comprise one or more colours, placed in a differentiated manner, which in turn are individually accommodated in cups made of metal or other material.

The aesthetic appearance of such a product plays a very important role, especially if "amazing" colour effects are successfully produced, which are generated by individual colours which in turn occupy various shapes enclosed in a single package.

Several processes are known, by which solid, "multicolour" beauty products are obtained, which in addition to have a primarily aesthetic purpose, are aimed at allowing consumers to obtain a make-up having colour shades derived by mixing the colours existing in the package, once the product has been applied by means of a specific accessory.

Some known processes include matching one or more colours, possibly divided by diaphragms which prevent the various colours from getting mixed.

It is the object of the invention to provide a different process as compared to those of the known art, which allows to amalgamate several colours having different geometrical shapes in a single package so as to create amazing aesthetic effects.

In accordance with the present invention, such an object is achieved by means of a process comprising the following steps in sequence:
a) pouring an amount of a first powder beauty product having a first colour into a rotating drum mixer and spraying it with an agglomerating liquid until base pellets consisting of said first beauty product are formed;
b) sprinkling said base pellets in said rotating drum mixer with an amount of a second powder beauty product having a second colour sprayed with said agglomerating liquid so as to obtain coated pellets consisting of said base pellets and a coating with said second powder beauty product;
c) possibly repeating step b) with further coating beauty products having further colours to obtain further coated pellets;
d) filling a container with a plurality of said coated pellets;
e) pre-compacting said plurality of coated pellets in said container;
f) cutting the pre-compacted pellets to highlight the colour differences between the base pellets and coatings thereof;
g) finally compacting said pre-compacted, cut pellets.

A solid, multicolour beauty product may be thus obtained, which is able to offer amazing colour effects capable of drawing the consumers' attention and inducing them to purchase it.

The various steps of the process according to the invention and the obtained results will be apparent form the following detailed description of a practical embodiment of the process itself, with reference to the accompanying drawings in which:
figures 1-8 show the various steps of the process according to the invention:
figure 9 shows a plan view of a wafer of the beauty product which may be obtained by the process according to the invention.

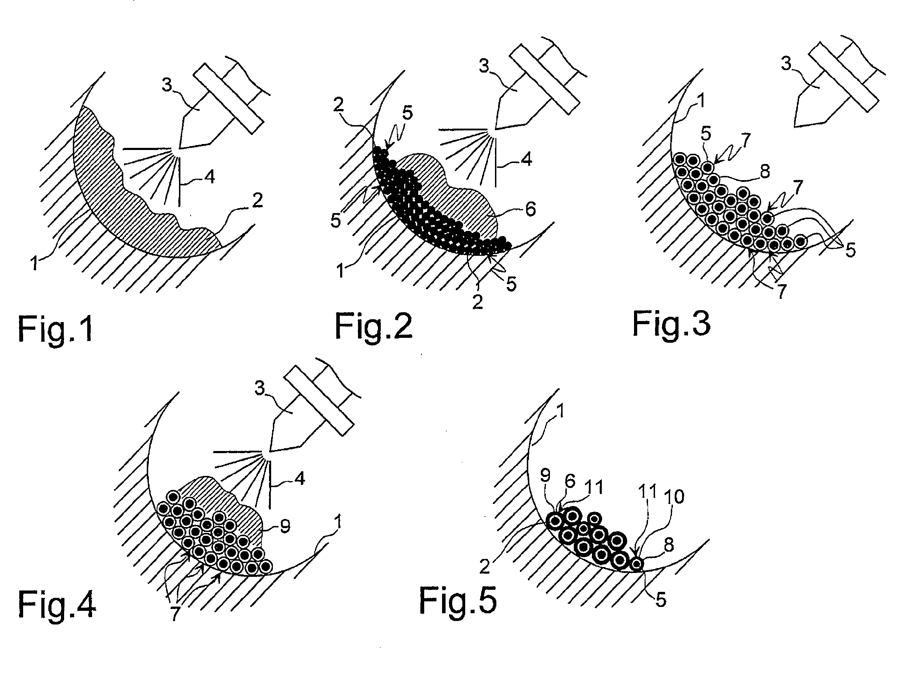

Figure 1 shows a rotating drum mixer 1 within which an amount of a first powder beauty product 2 having a fist colour is placed, which is sprayed with an agglomerating liquid product 4 by means of a vaporizer 3.
   Such a liquid may consist in 100% water or a mixture of water and various raw materials such as, for example, surfactants, emulsifiers or anything else may be used to promote the direct formation of pellets 5 from the powder 2 as it is, by means of the rotation of the drum mixer 1.
   Figure 2 shows drum mixer 1 containing pellets 5, which are sprinkled with a second powder beauty product 6 having a different colour as compared to the first, which is sprayed with agglomerating liquid with the aid of the vaporizer 3, the same liquid being used in the previous step, so as to coat pellets 5 with a layer of beauty powder having a second colour, generally different from that of the base pellet. Due to the rotation of drum mixer 1, coated pellets 7 are thus formed consisting of the base pellets 5 having a first colour and a coating 8 having a second colour, generally different from the first, as shown in figure 3.
   The size of base pellets and coating layer may vary very much depending on the needs.
   Figure 4 shows drum mixer 1 again, in which coated pellets 7 are sprinkled with a third powder beauty product 9, preferably having a different colour as compared to the previous two, in order to form a further coating 10.
   The coating treatment of pellets 7 with this further powder 9 occurs by employing vaporizer 3 which sprays the product with the agglomerating liquid, as previously disclosed.
   Figure 5 shows the thus obtained pellets, indicated by numeral 11 as a whole, consisting of a base pellet 5 of beauty product 2 and two external coatings 8, 10 of beauty products 6, 9, respectively.
   The number of coating layers may also be higher than three and exclusively depends on the size and colours to be obtained.

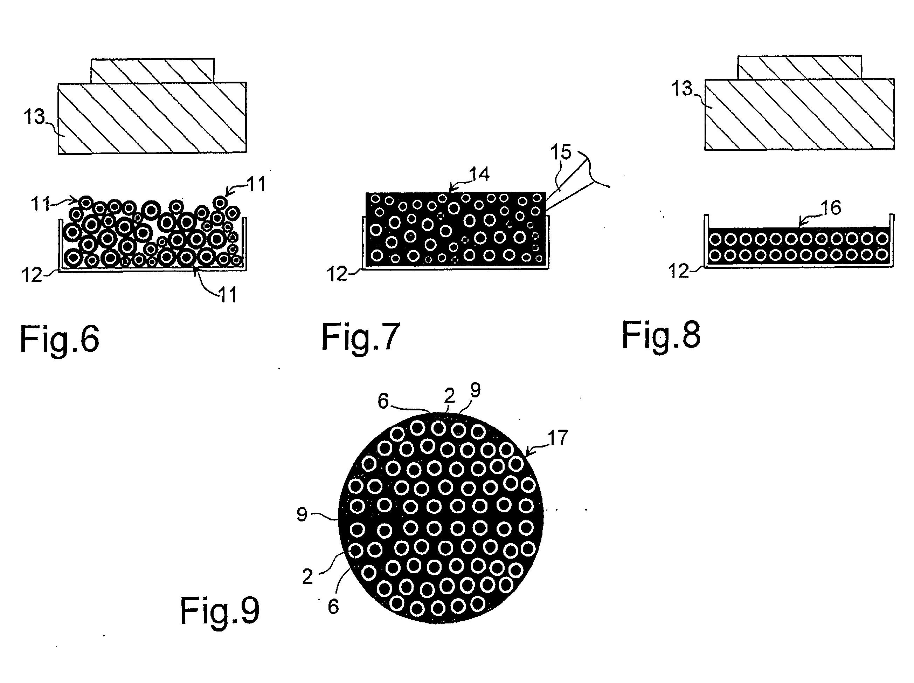

   Figure 6 shows a container or cup 12 which contains pellets 11 prior to be subjected to the pre-compacting treatment by means of a presser 13.
   Figure 7 shows pre-compacted product 14 which is cut by a blade 15 so that shape and colour differences may be highlighted and the final piece may be cut-to-size.
   Figure 8 shows container 12 containing final beauty product 16 after the final compacting treatment by presser 13.
   The result of the described process may be seen in figure 9, where a wafer 17 having a mosaic effect is shown, which consists of pellet-shaped bodies of first beauty product 2 with rounded coatings of second beauty product 6 and filling parts of beauty product 9.

## Claims

1. A process for producing powder wafers for eye shadows, face powder, make-up articles, blusher, etc., comprising the following steps in sequence:
a) pouring an amount of a first powder beauty product (2) having a first colour into a rotating drum mixer (1) and spraying it with an agglomerating liquid until base pellets (5) of said first beauty product (2) are formed;
b) sprinkling said base pellets (5) in said rotating drum mixer (1) with an amount of a second powder beauty product (6) having a second colour sprayed with an agglomerating liquid so as to obtain coated pellets (7) consisting of said base pellets (5) and a coating (8) with said second powder beauty product (6);
c) possibly repeating step b) with other coating beauty products (9) having further colours to obtain further coated pellets (11);
d) filling a container (12) with said coated pellets (7, 11);
e) pre-compacting said coated pellets (7, 11) in said container (12);
f) cutting pre-compacted pellets (7, 11) to highlight the colour differences between base pellets (5) and coatings (8, 10) thereof;
g) finally compacting said pre-compacted, cut pellets (7, 11).

2. A process according to claim 1, **characterized in that** said powder beauty products (2, 6, 9) have different colours.

3. A process according to claim 1, **characterized in that** said powder beauty products (2, 6, 9) have the same colour.

## Patentansprüche

1. Prozess zum Erzeugen von Pulverwafern für Lidschatten, Gesichtspulver, Make-up-Gegenstände, Rouge, etc., der die folgende Schrittfolge aufweist:
a) Gießen einer Menge eines ersten Pulverschönheitsproduktes (2), das eine erste Farbe hat, in einen drehenden Trommelmixer (1) und Besprühen desselben einer agglomerierenden Flüssigkeit, bis ein Basisgranulat (5) des ersten Schönheitsproduktes (2) ausgebildet wird;
b) Benetzen des Basisgranulats (5) in dem drehenden Trommelmixer (1) mit einer Menge eines zweiten Pulverschönheitsproduktes (6), das eine zweite Farbe hat, das mit einer agglomerierenden Flüssigkeit so besprüht wird, dass ein beschichtetes Granulat (7) erhalten wird, das aus dem Basisgranulat (5) und einer Beschichtung (8) mit dem zweiten Pulverschönheitsprodukt (6) besteht;
c) Mögliches Wiederholen des Schrittes b) mit anderen beschichtenden Schönheitsprodukten (9), die weitere Farben haben, um ein weiteres, beschichtetes Granulat (11) zu erhalten;
d) Füllen eines Behälters (12) mit dem beschichteten Granulat (7, 11);
e) Vor-Kompaktieren des beschichteten Granulats (7, 11) in dem Behälter (12);
f) Schneiden des vor-kompaktierten Granulats (7, 11), um die Farbdifferenzen zwischen dem Basisgranulat (5) und ihren Beschichtungen (8, 10) zu betonen;
g) Abschließendes Kompaktieren des vor-kompaktierten, geschnittenen Granulats(7, 11).

2. Prozess gemäß Anspruch 1, **dadurch gekennzeichnet, dass** die Pulverschönheitsprodukte (2, 6, 9) unterschiedliche Farben haben.

3. Prozess gemäß Anspruch 1, **dadurch gekennzeichnet, dass** die Pulverschönheitsprodukte (2, 6, 9) dieselbe Farbe haben.

## Revendications

1. Procédé de fabrication de wafers en poudre pour fards à paupières, poudre pour le visage, articles de maquillage, blush, etc., qui comprend les étapes suivantes, dans l'ordre :
a) le versement d'une quantité d'un premier produit de beauté en poudre (2) ayant une première couleur dans un mélangeur à tambour rotatif (1) et sa pulvérisation à l'aide d'un liquide antiagglomérant jusqu'à ce que des pastilles de base (5) dudit premier produit de beauté (2) soient formées ;
b) le saupoudrage desdites pastilles de base (5) dans ledit mélangeur à tambour rotatif (1) avec une quantité d'un second produit de beauté en poudre (6) ayant une seconde couleur et pulvérisé avec un liquide antiagglomérant de façon à obtenir des pastilles enrobées (7) composées desdites pastilles de base (5) et d'un enrobage (8) avec ledit second produit de beauté en poudre (6) ;
c) la répétition éventuelle de l'étape b) avec d'autres produits de beauté d'enrobage (9) ayant d'autres couleurs, afin d'obtenir d'autres pastilles enrobées (11) ;
d) le remplissage d'un conteneur (12) avec lesdites pastilles enrobées (7, 11) ;
e) le pré-compactage desdites pastilles enrobées (7, 11) dans ledit conteneur (12) ;
f) la découpe desdites pastilles pré-compactées (7, 11) afin de mettre en exergue les différences de couleur entre lesdites pastilles de base (5) et leurs enrobages (8, 10) ;
g) le compactage final desdites pastilles pré-compactées découpées (7, 11).

2. Procédé selon la revendication 1, **caractérisé en ce que** lesdits produits de beauté en poudre (2, 6, 9) possèdent des couleurs différentes.

3. Procédé selon la revendication 1, **caractérisé en ce que** lesdits produits de beauté en poudre (2, 6, 9) possèdent la même couleur.
